# EUROPÄISCHE PATENTSCHRIFT

(11) **EP 2 917 179 B1**
(45) Veröffentlichungstag und Bekanntmachung des Hinweises auf die Patenterteilung: **20.06.2018**
(21) Anmeldenummer: 13776762.0
(22) Anmeldetag: 09.10.2013
(51) Int. Cl.: C07C 407/00, C07C 409/26

(54) **VERFAHREN ZUR HERSTELLUNG VON GLEICHGEWICHTSPERESSIGSÄURE UND MIT DEM VERFAHREN ERHÄLTLICHE GLEICHGEWICHTSPERESSIGSÄURE**
METHOD FOR THE PREPARATION OF EQUILIBRIUM PERACETIC ACID AND EQUILIBRIUM PERACETIC ACID OBTAINABLE BY THE METHOD
PROCÉDÉ DE FABRICATION D'ACIDE PERACÉTIQUE À ÉQUILIBRE DE MASSE ET ACIDE PERACÉTIQUE À ÉQUILIBRE DE MASSE OBTENU PAR CE PROCÉDÉ

(30) Priorität: 08.11.2012 EP 12191799
(43) Veröffentlichungstag der Anmeldung: 16.09.2015
(73) Patentinhaber: Evonik Degussa GmbH, 45128 Essen (DE)
(72) Erfinder: REINOLD, Andreas, 63584 Gründau (DE); LEININGER, Stefan, 63505 Langenselbold (DE); HELLWIG, Angela, 63303 Dreieich (DE)
(86) Internationale Anmeldenummer: PCT/EP2013/071013
(87) Internationale Veröffentlichungsnummer: WO 2014/072143

(56) Entgegenhaltungen:
- EP-A1- 0 742 206
- WO-A1-01/29004
- WO-A1-03/051843
- US-A- 4 587 264

## Beschreibung

Die Erfindung betrifft eine Verfahren zur Herstellung von Gleichgewichtsperessigsäure und die mit dem Verfahren erhältliche Gleichgewichtsperessigsäure geringer Korrosivität.

Peroxyessigsäure findet Anwendung als ein Desinfektionsmittel, das schon bei niedrigen Temperaturen eine breite Wirkung gegen Bakterien und andere Mikroorganismen, sowie gegen Viren zeigt und gegen das Bakterien und Viren keine Resistenz bilden. Peroxyessigsäure hat dabei den Vorteil, dass bei den Anwendungskonzentrationen, die zur Desinfektion eingesetzt werden, nach einiger Zeit eine vollständige Hydrolyse zu Essigsäure und Wasserstoffperoxid erfolgt, sodass die biozide Wirkung verloren geht und es nicht zu einer Akkumulierung von Biozid kommen kann.

Peroxyessigsäure wird üblicherweise in Form von Gleichgewichtsperessigsäure in den Handel gebracht, die für die Anwendung mit Wasser auf die gewünschte Anwendungskonzentration gebracht wird. Als Gleichgewichtsperessigsäure wird dabei eine Mischung bezeichnet, die Peroxyessigsäure, Wasserstoffperoxid, Essigsäure und Wasser im chemischen Gleichgewicht enthält. Das chemische Gleichgewicht stellt sich dabei zwischen diesen vier Komponenten entsprechend folgender Reaktionsgleichung ein:

CH₃C(O)OH + H₂O₂ ⇆ CH₃C(O)OOH + H₂O

Gleichgewichtsperessigsäure wird üblicherweise aus Essigsäure und wässriger Wasserstoffperoxidlösung durch Einstellung dieses Gleichgewichts hergestellt. Die Einstellung des Gleichgewichts erfolgt in Abwesenheit weiterer Komponenten allerdings nur langsam und erfordert bei 20 °C mehrere Tage. Um die Einstellung des Gleichgewichts zu beschleunigen wird deshalb üblicherweise Schwefelsäure als Katalysator zugesetzt. Ein Zusatz von größeren Mengen an Schwefelsäure ermöglicht außerdem in Desinfektionsanwendungen die Regelung der dosierten Menge an Peroxyessigsäure über die Leitfähigkeit der Desinfektionslösung.

Gleichgewichtsperessigsäure die Schwefelsäure enthält hat den Nachteil, dass sie auf metallische Werkstoffe korrosiv wirkt und dass sie in Kontakt mit Edelstahl nicht lagerfähig ist, da aus dem Werkstoff herausgelöste Eisenionen die Zersetzung von Peroxyessigsäure katalysieren.

In EP 0 087 343 wird vorgeschlagen, Salpetersäure als Katalysator für die Herstellung von Gleichgewichtsperessigsäure zu verwenden. Damit lässt sich zwar die Lochfraßkorrosion an Edelstahl verringern, doch werden durch solche Gleichgewichtsperessigsäure immer noch so viel Eisenionen aus Edelstahl herausgelöst, dass die Gleichgewichtsperessigsäure in Kontakt mit Edelstahl nicht lagerstabil ist.

In EP 0 742 206 A1 wird vorgeschlagen, Polyphosphorsäure als Katalysator für die Herstellung von Gleichgewichtsperessigsäure zu verwenden. Durch einen Zusatz von 0,2 bis 10 Gew.-% Polyphosphorsäure wird eine Gleichgewichtsperessigsäure erhalten, die in Gegenwart von Edelstahl lagerstabil ist. Nachteilig ist allerdings, dass Polyphosphorsäure sehr viskos ist und deshalb bei der Herstellung der Gleichgewichtsperessigsäure schwierig zu dosieren ist. Außerdem kann der Gehalt an Polyphosphorsäure bei der Behandlung von großen Wasservolumina, wie der Desinfektion von Klärwerksabläufen oder der Ballastwasserbehandlung von Hochseeschiffen, zu einer unerwünschten Eutrophierung von Gewässern führen.

Es besteht demnach weiterhin ein Bedarf für ein Verfahren zur Herstellung von Gleichgewichtsperessigsäure durch Umsetzung von Essigsäure mit Wasserstoffperoxid, das nicht den Nachteil des aus EP 0 742 206 A1 bekannten Verfahrens aufweist und eine Gleichgewichtsperessigsäure liefert, die in Gegenwart von Edelstahl lagerstabil ist und nicht eutrophierend wirkt.

JP 10-330357 beschreibt eine Herstellung von Peroxyessigsäure durch Umsetzung von Butylacetat mit 60 Gew.-% Wasserstoffperoxidlösung unter Zusatz von Methansulfonsäure als Katalysator. Durch die Umsetzung in Abwesenheit einer Carbonsäure sollen Korrosionsprobleme vermieden werden.

Das Gebrauchsmuster DE 20 2007 005 732 U1 beschreibt ein Reinigungsmittel zur Beseitigung fester Ablagerungen in Trinkwasserleitungen, das 1 bis 14 Gew.-% Amidosulfonsäure, 5 bis 10 Gew.-% Methansulfonsäure, 0,075 bis 0,15 Gew.-% Phosphonobutantricarbonsäure, 0,3 Gew.-% 2-Propanol und zu 100 % ergänzend Wasser enthält. Das Reinigungsmittel kann durch Zugabe von 1 bis 10 Gew.-% Desinfektionsmittel mit einer Desinfektionswirkung versehen sein, wobei als Desinfektionsmittel wasserstoffperoxidhaltige Stoffe, wie z.B. Peressigsäure eingesetzt werden können.

WO 2012/001276 beschreibt Zusammensetzungen für die Auflösung von Oxalatablagerungen, die Methansulfonsäure und zusätzlich Phosphorsäure oder Salpetersäure enthalten. Die Zusammensetzungen können ein oder mehrere Additive enthalten, wobei unter anderem als Desinfektionsmittel Bromessigsäure, Peressigsäure, Salicylsäure und Wasserstoffperoxid genannt werden.

Es wurde nun überraschend gefunden, dass sich die oben genannten Aufgaben lösen lassen, wenn zur Herstellung von Gleichgewichtsperessigsäure aus Essigsäure und Wasserstoffperoxid Methansulfonsäure als Katalysator verwendet wird.

Gegenstand der Erfindung ist deshalb ein Verfahren zur Herstellung von Gleichgewichtsperessigsäure durch Umsetzung von Essigsäure mit Wasserstoffperoxid in wässriger Reaktionsmischung, wobei die Umsetzung in Gegenwart von Methansulfonsäure als Katalysator durchgeführt wird.

Gegenstand der Erfindung ist außerdem die Verwendung von Methansulfonsäure als Katalysator zur Herstellung von Gleichgewichtsperessigsäure aus Essigsäure und Wasserstoffperoxid.

Ein weiterer Gegenstand der Erfindung ist eine Gleichgewichtsperessigsäure, die Peroxyessigsäure, Wasserstoffperoxid, Essigsäure und Wasser umfasst und Methansulfonsäure enthält.

Bei dem erfindungsgemäßen Verfahren werden Essigsäure und Wasserstoffperoxid in wässriger Reaktionsmischung in Gegenwart von Methansulfonsäure zu Gleichgewichtsperessigsäure umgesetzt. Methansulfonsäure wirkt dabei als Katalysator, der die Geschwindigkeit der Reaktion von Essigsäure mit Wasserstoffperoxid zu Peroxyessigsäure erhöht. Die Umsetzung wird vorzugsweise durchgeführt, bis der Gehalt an Peroxyessigsäure mehr als 90 % und besonders bevorzugt mehr als 95 % des im chemischen Gleichgewicht vorliegenden Gehalts erreicht hat.

In einer bevorzugten Ausführungsform des erfindungsgemäßen Verfahrens enthält die Reaktionsmischung zur Umsetzung von Essigsäure mit Wasserstoffperoxid 0,1 bis 2,0 Gew.-% Methansulfonsäure, besonders bevorzugt 0,5 bis 2,0 Gew.-% Methansulfonsäure. Durch die Verwendung von Methansulfonsäure in diesem Konzentrationsbereich lässt sich einerseits eine weitgehende Einstellung des Gleichgewichts in ausreichend kurzer Zeit erreichen und andererseits eine Gleichgewichtsperessigsäure erhalten, die eine geringe Korrosionswirkung auf Metalle und eine geringe Zersetzung bei Kontakt mit Metallen aufweist.

In einer weiteren bevorzugten Ausführungsform des erfindungsgemäßen Verfahrens enthält die Reaktionsmischung zur Umsetzung von Essigsäure mit Wasserstoffperoxid 5 bis 30 Gew.-% Methansulfonsäure. Durch die Verwendung von Methansulfonsäure in diesem Konzentrationsbereich lässt sich eine Gleichgewichtsperessigsäure erhalten, deren Dosierung für Desinfektionsanwendungen über die elektrische Leitfähigkeit der verdünnten Lösung geregelt werden kann und die gegenüber einer Gleichgewichtsperessigsäure, die für den gleichen Zweck geeignete Mengen an Schwefelsäure enthält, eine wesentlich geringere Korrosionswirkung auf Metalle aufweist. Außerdem ist in dieser Ausführungsform die Wärmeentwicklung in der Reaktionsmischung deutlich geringer als bei Verwendung einer entsprechenden Menge an Schwefelsäure.

Für das erfindungsgemäße Verfahren kann Essigsäure in reiner Form oder in Form einer wässrigen Lösung eingesetzt werden. Vorzugsweise wird Essigsäure in reiner Form eingesetzt. Wasserstoffperoxid wird vorzugsweise in Form einer wässrigen Lösung mit einem Gehalt von 10 bis 85 Gew.-%, besonders bevorzugt 25 bis 60 Gew.-% eingesetzt. Für die Umsetzung kann der Reaktionsmischung optional noch Wasser zugesetzt werden. Vorzugsweise enthält die wässrige Reaktionsmischung kein zusätzliches Lösungsmittel neben Wasser. Die Mengen an Essigsäure, Wasserstoffperoxid und Wasser in der Reaktionsmischung werden so gewählt, dass nach Einstellung des Gleichgewichts der gewünschte Gehalt an Peroxyessigsäure und Wasserstoffperoxid in der Gleichgewichtsperessigsäure erreicht wird. Essigsäure und Wasserstoffperoxid werden dazu vorzugsweise in einem molaren Verhältnis im Bereich von 0,5 bis 10, besonders bevorzugt 0,5 bis 5 eingesetzt.

Methansulfonsäure wird für das erfindungsgemäße Verfahren vorzugsweise in reiner Form oder in Form einer wässrigen Lösung eingesetzt, besonders bevorzugt in Form einer wässrigen Lösung, die 1 bis 40 Gew.-% Wasser enthält. In einer bevorzugten Ausführungsform des erfindungsgemäßen Verfahrens wird die Reaktionsmischung durch Mischen von Essigsäure, wässriger Wasserstoffperoxidlösung mit einem Gehalt an 25 bis 60 Gew.-% Wasserstoffperoxid und Methansulfonsäure hergestellt, wobei die Methansulfonsäure 1 bis 40 Gew.-% Wasser enthalten kann.

Die Reihenfolge, in der Essigsäure, Wasserstoffperoxid und Methansulfonsäure der Reaktionsmischung zugegeben werden, ist für das erfindungsgemäße Verfahren nicht wesentlich. Vorzugsweise wird jedoch Essigsäure vorgelegt und Wasserstoffperoxid zudosiert, um eine Bildung von Mischungen zu vermeiden, bei denen es durch eine selbstbeschleunigende Zersetzung zu hohen Druckanstiegsgeschwindigkeiten kommen kann. Methansulfonsäure wird vorzugsweise nach Mischen von Essigsäure und Wasserstoffperoxid zugegeben oder einem der Einsatzstoffe Essigsäure oder wässrige Wasserstoffperoxidlösung zugesetzt. Besonders bevorzugt wird die Methansulfonsäure der Wasserstoffperoxidlösung zugesetzt.

Zur Herstellung der wässrigen Reaktionsmischung können im erfindungsgemäßen Verfahren Essigsäure und wässrige Wasserstoffperoxidlösung ansatzweise miteinander gemischt werden. Alternativ können Essigsäure und wässrige Wasserstoffperoxidlösung auch kontinuierlich gemischt werden, beispielsweise über einen statischen Mischer. Für ein kontinuierliches Mischen kann das in WO 02/26459 beschriebene Verfahren eingesetzt werden. Nach dem Mischen von Essigsäure und wässriger Wasserstoffperoxidlösung kann die Umsetzung zu Gleichgewichtsperessigsäure in einem beliebigen Behälter erfolgen. Da die Reaktionswärme der Umsetzung gering ist, kann die Umsetzung ohne Kühlung und sogar adiabatisch erfolgen. In einer bevorzugten Ausführungsform erfolgt die Umsetzung nach dem Mischen in einem Lagerbehälter oder Transportbehälter für Gleichgewichtsperessigsäure.

Die mit dem erfindungsgemäßen Verfahren erhältliche erfindungsgemäße Gleichgewichtsperessigsäure umfasst Peroxyessigsäure, Wasserstoffperoxid, Essigsäure, Wasser und Methansulfonsäure. Peroxyessigsäure, Wasserstoffperoxid, Essigsäure und Wasser stehen dabei im chemischen Gleichgewicht. Vorzugsweise enthält die erfindungsgemäße Gleichgewichtsperessigsäure 2 bis 24 Gew.-%, besonders bevorzugt 3 bis 17 Gew.-% und insbesondere 4 bis 15 Gew.-% Peroxyessigsäure.

In einer bevorzugten Ausführungsform enthält die erfindungsgemäße Gleichgewichtsperessigsäure 2 bis 12 Gew.-% Peroxyessigsäure und 5 bis 30 Gew.-% Methansulfonsäure und das Gewichtsverhältnis von Methansulfonsäure zu Peroxyessigsäure liegt im Bereich von 2 bis 5. In dieser Ausführungsform kann das Verdünnen der Gleichgewichtsperessigsäure auf die Anwendungskonzentration für Desinfektionsanwendungen durch die elektrische Leitfähigkeit der verdünnten Lösung geregelt werden.

In einer weiteren bevorzugten Ausführungsform enthält die erfindungsgemäße Gleichgewichtsperessigsäure zusätzlich zu Peroxyessigsäure, Wasserstoffperoxid, Essigsäure, Wasser und Methansulfonsäure noch bis zu 5 Gew.-% an einem oder mehreren Tensiden. Geeignete Tenside zur Verwendung in Gleichgewichtsperessigsäure sind aus dem Stand der Technik bekannt, beispielsweise aus DE 26 16 049 A1, EP 147 207, WO 93/10088, EP 873 687 und WO 98/37762. Bevorzugte Tenside sind anionische Tenside, wie Alkylsulfonate, Alkylsulfate und Alkylbenzolsulfonate, und nichtionische Tenside, wie Fettalkoholalkoxylate, Fettsäurealkoxylate und tertiäre Aminoxide mit einem langkettigen Alkylrest. Durch den Zusatz von Tensiden lassen sich Gleichgewichtsperessigsäuren herstellen, die auch nach Verdünnen auf die Anwendungskonzentration für Desinfektionsanwendungen auf einer zu desinfizierenden Oberfläche spreiten und diese vollständig benetzen. Dadurch lässt sich bei einem Aufsprühen der verdünnten Lösung auf eine Oberfläche sicherstellen, dass die Oberfläche lückenlos mit Peroxyessigsäure behandelt wird.

Die erfindungsgemäße Gleichgewichtsperessigsäure kann zusätzlich noch einen oder mehrere Stabilisatoren enthalten, die Peroxyessigsäure gegen eine katalytische Zersetzung durch Schwermetallionen stabilisieren. Geeignete Stabilisatoren für Peroxyessigsäure sind aus dem Stand der Technik bekannt, beispielsweise aus EP 742 206 B1, Absätze [0016] und [0017]. Bevorzugte Stabilisatoren sind 1-Hydroxyethan-1,1-diphosphonsäure, Aminotrimethylenphosphonsäure, Ethylendiamintetra(methylenphosphonsäure), Dipicolinsäure und deren Alkalimetallsalze.

Die erfindungsgemäße Gleichgewichtsperessigsäure kann zusätzlich noch eine oder mehrere härtestabilisierende Verbindungen enthalten, die Calcium- und Magnesiumionen komplexieren können. Geeignete härtestabilisierende Verbindungen für Gleichgewichtsperessigsäure sind aus dem Stand der Technik bekannt, beispielsweise aus EP 945 405 B1, Absatz [0005]. Vorzugsweise wird als härtestabilisierende Verbindung ein Carboxylgruppen enthaltendes Polymer verwendet. Besonders bevorzugt sind Polymere aus der Reihe (i) durch oxidative Polymerisation von Acrolein oder von Acrolein und Acrylsäure hergestellte Polymere, (ii) Polyacrylsäure, (iii) Copolymere aus Acrylsäure und einer anderen ungesättigten Carbonsäure, insbesondere Maleinsäure, und (iv) Polymaleinsäure, wobei das mittlere Molekulargewicht Mw des Polymers im Bereich von 500 bis 25000, insbesondere 1000 bis 15000, liegt. In der Gleichgewichtsperessigsäure können Carboxylgruppen des härtestabilisierenden Polymers teilweise in Percarboxylgruppen überführt sein.

In einer bevorzugten Ausführungsform enthält die erfindungsgemäße Gleichgewichtsperessigsäure weniger als 1 Gew.-% zusätzliche Verbindungen neben Peroxyessigsäure, Wasserstoffperoxid, Essigsäure, Wasser, Methansulfonsäure, optional bis zu 5 Gew.-% Tenside und optional bis zu 10 Gew.-% Carboxylgruppen enthaltenden Polymeren.

Die nachfolgenden Beispiele erläutern die Erfindung, ohne jedoch den Gegenstand der Erfindung zu beschränken.

### Beispiele

### Beispiele 1 bis 5

### Geschwindigkeit der Gleichgewichtseinstellung für eine 15 Gew.-% Gleichgewichtsperessigsäure

Eine Mischung aus 28,2 Gew.-% Wasserstoffperoxid, 28,4 Gew.-% Essigsäure, dem in Tabelle 1 angeführten Gewichtsanteil Katalysator und im Rest Wasser wurde bei 20 °C gehalten und die Zunahme des Gehalts an Peroxyessigsäure mit der Zeit wurde durch Redoxtitration verfolgt, bis der Gehalt an Peroxyessigsäure konstant blieb, d.h., das Gleichgewicht erreicht war. Dazu wurden Proben genommen, das darin enthaltene Wasserstoffperoxid durch rasche Titration mit Ce(IV)sulfat und Ferroin-Indikator umgesetzt, unmittelbar anschließend mit einem Überschuss Kaliumjodid versetzt und das durch Reaktion mit Peroxyessigsäure freigesetzte Jod mit Thiosulfat und Stärkeindikator titriert. Die Zunahme der Peroxyessigsäurekonzentration wurde nach einer Kinetik pseudoerster Ordnung ausgewertet. Dazu wurde für die Messwerte der ersten 8 h In[(c_{G}-c)/c_{G]} gegen die Reaktionszeit t aufgetragen, wobei c_{G} die PeroxyessigsäureKonzentration im Gleichgewicht und c die PeroxyessigsäureKonzentration zum Zeitpunkt t ist. Die Auftragung ergibt eine Gerade, aus deren Steigung die in Tabelle 1 zusammengestellten Zeiten t₈₈ bis zum Erreichen von 88 % der Gleichgewichtskonzentration berechnet wurden. Diese Auswertung nach einer Kinetik pseudoerster Ordnung unterschätzt die Dauer bis zum Erreichen von 88 % der Gleichgewichtskonzentration um etwa den Faktor 1,3.

**Tabelle 1**

| Geschwindigkeit der Gleichgewichtseinstellung | | |
|---|---|---|
| Beispiel | Katalysator | t₈₈ in h |
| 1* | 1,0 Gew.-% H₂SO₄ | 18 |
| 2* | 2,0 Gew.-% Polyphosphorsäure | 16 |
| 3 | 0,5 Gew.-% CH₃SO₃H | 38 |
| 4 | 1,0 Gew.-% CH₃SO₃H | 20 |
| 5 | 1,5 Gew.-% CH₃SO₃H | 14 |

| | | |
|---|---|---|
| *nicht erfindungsgemäß | | |

### Beispiel 6

### Lagerstabilität bei Kontakt mit Edelstahl

Eine Mischung aus 1275,5 g 49,4 Gew.-% Wasserstoffperoxid PERSYNTH® 500LC, 1171,2 g Essigsäure, 530,2 g vollentsalztem Wasser, 21,0 g Methansulfonsäure, 2,0 g 1-Hydroxyethan-1,1-diphosphonsäure und 0,3 g Pyridin-2,6-dicarbonsäure wurde durch Stehenlassen bei Zimmertemperatur zur Gleichgewichtsperessigsäure umgesetzt. Die gebildete Gleichgewichtsperessigsäure enthielt 15,2 Gew.-% Peroxyessigsäure, 14,3 Gew.-% Wasserstoffperoxid und 0,7 Gew.-% Methansulfonsäure. Für diese Gleichgewichtsperessigsäure wurde mit dem Prüfverfahren UN H.2 für gefährliche Güter der UN-Klasse 5.2 in einem adiabatischen Warmlagerversuch in einem 1 l Kugeldewargefäß unter Zusatz eines gebeizten Metallcoupons eine selbstbeschleunigende Zersetzungstemperatur (SADT) von 50 °C für eine Lagerung in einem nicht isolierten 17,5 m³ Edelstahltank bestimmt.

### Beispiel 7

### Lagerstabilität bei hohem Gehalt an Methansulfonsäure

Für eine Gleichgewichtsperessigsäure, enthaltend 5,3 Gew.-% Peroxyessigsäure, 23,7 Gew.-% Wasserstoffperoxid, 4,8 Gew.-% Essigsäure, 15,0 Gew.-% Methansulfonsäure, 0,04 Gew.-% 1-Hydroxyethan-1,1-diphosphonsäure, 0,01 Gew.-% Pyridin-2,6-dicarbonsäure und 51,1 Gew.-% Wasser, wurde mit dem Prüfverfahren UN H.4 für gefährliche Güter der UN-Klasse 5.2 in einem isoperibolen Warmlagerversuch bei 55 °C in einem 0,5 l zylindrischen Dewargefäß eine selbstbeschleunigende Zersetzungstemperatur (SADT) von mindestens 60 °C für eine Lagerung in einem 50 kg Gebinde bestimmt.

Für die gleiche Gleichgewichtsperessigsäure wurde mit dem Prüfverfahren UN H.2 für gefährliche Güter der UN-Klasse 5.2 in einem überadiabatischen Lagertest mit 1 l Probe und zwei HDPE-Plättchen der Abmessungen 20*60*2 mm in einem sphärischen 1 l Dewargefäß eine selbstbeschleunigende Zersetzungstemperatur (SADT) von 70 °C für eine Lagerung in einem 1 m³ HDPE-IBC bestimmt.

Für eine Gleichgewichtsperessigsäure mit der gleichen Zusammensetzung wurden die Prüfungen entsprechend Sektion 20, Fließdiagramm von Fig. 20.1 der UN Recommendations on the Transport of Dangerous Goods, Manual of Tests and Criteria durchgeführt und eine Einstufung als organisches Peroxid Typ F erhalten, d.h. für die Gleichgewichtsperessigsäure kommt ein Transport in IBCs oder Tankbehältern in Frage.

### Beispiele 8 bis 11

### Korrosionswirkung von verdünnten Lösungen und Langzeitlagerestabilität

In Beispiel 8 wurden 1141,0 g 49,4 Gew.-% Wasserstoffperoxid PERSYNTH® 500LC, 567,6 g Essigsäure, 269,9 g vollentsalztes Wasser, 19,8 g 98 Gew.-% Schwefelsäure, 1,6 g 1-Hydroxyethan-1,1-diphosphonsäure und 0,24 g Pyridin-2,6-dicarbonsäure gemischt und durch Stehenlassen bei Zimmertemperatur zur Gleichgewichtsperessigsäure umgesetzt. Die gebildete Gleichgewichtsperessigsäure enthielt 14,5 Gew.-% Peroxyessigsäure, 21,4 Gew.-% Wasserstoffperoxid und 1,0 Gew.-% Schwefelsäure. 3 Teile der Gleichgewichtsperessigsäure wurden mit 997 Teilen vollentsalztem Wasser zu einer Desinfektionslösung verdünnt. Die so erhaltene Desinfektionslösung wurde in Kontakt mit einem Werkstoffcoupon (Stahl Werkstoffnummer 1.0038 mit den Abmessungen 60*20*3 mm), der vorher 1 h bei 20 °C mit inhibierter Salzsäure gebeizt wurde, bei Zimmertemperatur gelagert. Nach 24 h war der Werkstoffcoupon deutlich angerostet und die Lösung gelblich verfärbt, der Gehalt an Peroxyessigsäure hatte auf 2 ppm (bestimmt mit Merck Reflectoquant®-Teststäbchen) abgenommen. Aus der Gewichtsabnahme des Werkstoffcoupons wurde ein Materialabtrag von 0,4 mm/a berechnet. Ohne Werkstoffcoupon lag der Gehalt an Peroxyessigsäure nach 24 h bei 380 ppm und nach 72 h bei 370 ppm (colorimetrisch bestimmt mit ABTS-Farbreagenz).

In Beispiel 9 wurde Beispiel 8 wiederholt, es wurden jedoch an Stelle der Schwefelsäure 28,6 g 70 Gew.-% Methansulfonsäure Lutropur MSA der Firma BASF eingesetzt und die Menge an vollentsalztem Wasser auf 261,2 g verringert. Die gebildete Gleichgewichtsperessigsäure enthielt 14,6 Gew.-% Peroxyessigsäure, 21,6 Gew.-% Wasserstoffperoxid und 1,0 Gew.-% Methansulfonsäure. Die durch Verdünnen hergestellte Desinfektionslösung zeigte bei der Lagerung in Kontakt mit dem Werkstoffcoupon keine Verfärbung, auch am Werkstoffcoupon war nach 72 h keine Veränderung sichtbar. Aus der Gewichtsabnahme des Werkstoffcoupons wurde ein Materialabtrag von weniger als 0,01 mm/a berechnet. Der Gehalt an Peroxyessigsäure lag bei Kontakt mit dem Werkstoffcoupon nach 24 h bei 380 ppm und nach 72 h bei 350 ppm und ohne Werkstoffcoupon nach 24 h bei 380 ppm und nach 72 h bei 360 ppm.

In Beispiel 10 wurden 1052,7 g 49,4 Gew.-% Wasserstoffperoxid PERSYNTH® 500LC, 160,1 g Essigsäure, 479,7 g vollentsalztes Wasser, 306,2 g 98 Gew.-% Schwefelsäure, 1,4 g 11-Hydroxyethan-1,1-diphosphonsäure und 0,18 g Pyridin-2,6-dicarbonsäure gemischt und durch Stehenlassen bei Zimmertemperatur zur Gleichgewichtsperessigsäure umgesetzt. Die gebildete Gleichgewichtsperessigsäure enthielt 5,0 Gew.-% Peroxyessigsäure, 23,7 Gew.-% Wasserstoffperoxid und 15,0 Gew.-% Schwefelsäure. 1 Teil der Gleichgewichtsperessigsäure wurde mit 99 Teilen vollentsalztem Wasser zu einer Desinfektionslösung verdünnt. Die so erhaltene Desinfektionslösung wurde wie in Beispiel 8 in Kontakt mit einem Werkstoffcoupon gelagert. Schon nach 15 min war der Werkstoffcoupon deutlich angerostet, die Lösung war nach 24 h gelb verfärbt und trüb, der Gehalt an Peroxyessigsäure hatte auf 7 ppm (bestimmt mit Merck Reflectoquant®-Teststäbchen) abgenommen. Aus der Gewichtsabnahme des Werkstoffcoupons wurde ein Materialabtrag von 8 mm/a berechnet. Ohne Werkstoffcoupon lag der Gehalt an Peroxyessigsäure nach 24 h bei 370 ppm (colorimetrisch bestimmt mit ABTS-Farbreagenz).

In Beispiel 11 wurde Beispiel 10 wiederholt, es wurden jedoch an Stelle der Schwefelsäure 428,7 g 70 Gew.-% Methansulfonsäure Lutropur MSA der Firma BASF eingesetzt und die Menge an vollentsalztem Wasser auf 357,3 g verringert. Die gebildete Gleichgewichtsperessigsäure enthielt 4,7 Gew.-% Peroxyessigsäure, 23,9 Gew.-% Wasserstoffperoxid und 15,0 Gew.-% Methansulfonsäure. Die durch Verdünnen hergestellte Desinfektionslösung zeigte nach 48 h Lagerung in Kontakt mit dem Werkstoffcoupon keine Verfärbung oder Trübung, auch am Werkstoffcoupon war nach 48 h keine Veränderung sichtbar. Aus der Gewichtsabnahme des Werkstoffcoupons wurde ein Materialabtrag von 2,7 mm/a berechnet. Der Gehalt an Peroxyessigsäure lag bei Kontakt mit dem Werkstoffcoupon nach 24 h bei 270 ppm und nach 48 h bei 80 ppm und ohne Werkstoffcoupon nach 24 h bei 360 ppm und nach 48 h bei 300 ppm.

Für die unverdünnten Gleichgewichtsperessigsäuren wurde außerdem die Lagerstabilität bei einer Lagerung bei Zimmertemperatur über einen Zeitraum von 86 Wochen bestimmt, indem jeweils Proben entnommen wurden und die Gehalte an Peressigsäure (PES) und Wasserstoffperoxid wie in den Beispielen 1 bis 5 bestimmt wurden. Aus den so bestimmten Gehalten an Peressigsäure (PES) und Wasserstoffperoxid wurde der Gesamtgehalt an Aktivsauerstoff (AO-Gehalt) berechnet. Die Ergebnisse sind in Tabelle 2 zusammengefasst.

**Tabelle 2**

| Langzeitlagerestabilität | | | | |
|---|---|---|---|---|
| Beispiel | Lagerdauer [Wochen] | Gehalt PES [ ]Gew.-%] | Gehalt H₂O₂ [Gew.-%] | AO-Gehalt [Gew.-%] |
| 8* | 1 | 14,6 | 21,6 | 13,2 |
| | 3 | 14,6 | 21,6 | 13,2 |
| | 7 | 14,6 | 21,6 | 13,2 |
| | 16 | 14,6 | 21,6 | 13,2 |
| | 26 | 14,5 | 21,4 | 13,1 |
| | 86 | 12,8 | 20,2 | 12,2 |
| 9 | 1 | 14,6 | 21,6 | 13,2 |
| | 3 | 14,6 | 21,6 | 13,2 |
| | 7 | 14,6 | 21,6 | 13,2 |
| | 16 | 14,6 | 21,6 | 13,2 |
| | 26 | 14,5 | 21,5 | 13, 2 |
| | 86 | 13,6 | 20,9 | 12, 7 |
| 10* | 1 | 5,1 | 23,7 | 12,2 |
| | 3 | 5,0 | 23,6 | 12,1 |
| | 7 | 5,0 | 23,6 | 12,1 |
| | 16 | 4,9 | 2,3 | 12,0 |
| | 26 | 4,9 | 23,1 | 11,9 |
| | 86 | 3,9 | 21,6 | 11,0 |
| 11 | 1 | 4,7 | 23,9 | 12,2 |
| | 3 | 4,7 | 23,9 | 12,2 |
| | 7 | 4,7 | 23,9 | 12,2 |
| | 16 | 4,7 | 23,8 | 12,1 |
| | 26 | 4,7 | 23,9 | 12,2 |
| | 86 | 4,3 | 24,0 | 12,2 |

| | | | | |
|---|---|---|---|---|
| * Nicht erfindungsgemäß | | | | |

Die Beispiele zeigen, dass die erfindungsgemäßen Gleichgewichtsperessigsäuren eine geringere Korrosivität und eine bessere Lagerstabilität aufweisen als vergleichbare Gleichgewichtsperessigsäuren, die Schwefelsäure ans Stelle von Methansulfonsäure enthalten.

Für die in den Beispielen 10 und 11 erhaltenen Gleichgewichtsperessigsäuren wurde außerdem die elektrische Leitfähigkeit bei 25 °C unverdünnt und nach Verdünnen mit vollentsalztem Wasser um die Faktoren 10, 100 und 1000 mit einem Konduktometer WTW 340i mit Elektrode Tetracon 325 bestimmt. Die Ergebnisse sind in Tabelle 3 zusammengefasst.

**Tabelle 3**

| Elektrische Leitfähigkeit | | |
|---|---|---|
| Beispiel | 10* | 11 |

| Elektrische Leitfähigkeit in mS/cm: | | |
|---|---|---|
| unverdünnt | 300 | 200 |
| 10-fach verdünnt | 65 | 53 |
| 100-fach verdünnt | 8,5 | 5,8 |
| 1000-fach verdünnt | 1,25 | 0,74 |

| | | |
|---|---|---|
| *nicht erfindungsgemäß | | |

## Patentansprüche

1. Verfahren zur Herstellung von Gleichgewichtsperessigsäure durch Umsetzung von Essigsäure mit Wasserstoffperoxid in wässriger Reaktionsmischung,
**dadurch gekennzeichnet,**
**dass** die Umsetzung in Gegenwart von Methansulfonsäure als Katalysator durchgeführt wird.

2. Verfahren nach Anspruch 1,
**dadurch gekennzeichnet,**
**dass** die Reaktionsmischung 0,1 bis 2,0 Gew.-% Methansulfonsäure enthält.

3. Verfahren nach Anspruch 1 oder 2,
**dadurch gekennzeichnet,**
**dass** die Reaktionsmischung kein zusätzliches Lösungsmittel enthält

4. Verfahren nach einem der Ansprüche 1 bis 3,
**dadurch gekennzeichnet,**
**dass** die Reaktionsmischung durch Mischen von Essigsäure, wässriger Wasserstoffperoxidlösung mit einem Gehalt an 25 bis 60 Gew.-% Wasserstoffperoxid und Methansulfonsäure hergestellt wird, wobei die Methansulfonsäure 1 bis 40 Gew.-% Wasser enthalten kann.

5. Verfahren nach einem der Ansprüche 1 bis 4,
**dadurch gekennzeichnet,**
**dass** Essigsäure und Wasserstoffperoxid in einem molaren Verhältnis im Bereich von 0,5 bis 10 eingesetzt werden.

6. Verfahren nach einem der Ansprüche 1 bis 5,
**dadurch gekennzeichnet,**
**dass** die Umsetzung durchgeführt wird bis der Gehalt an Peroxyessigsäure mehr als 90 % des im chemischen Gleichgewicht vorliegenden Gehalts erreicht hat.

7. Verwendung von Methansulfonsäure als Katalysator zur Herstellung von Gleichgewichtsperessigsäure aus Essigsäure und Wasserstoffperoxid.

8. Gleichgewichtsperessigsäure umfassend Peroxyessigsäure, Wasserstoffperoxid, Essigsäure und Wasser,
**dadurch gekennzeichnet,**
**dass** sie Methansulfonsäure enthält.

9. Gleichgewichtsperessigsäure nach Anspruch 8, **dadurch gekennzeichnet,**
**dass** sie 0 bis 5 Gew.-% Tenside, 0 bis 10 Gew.-% Carboxylgruppen enthaltende Polymere und weniger als 1 Gew.-% zusätzliche Verbindungen enthält.

10. Gleichgewichtsperessigsäure nach Anspruch 8 oder 9,
**dadurch gekennzeichnet,**
**dass** sie 3 bis 17 Gew.-% Peroxyessigsäure enthält.

11. Gleichgewichtsperessigsäure nach Anspruch 8 oder 9,
**dadurch gekennzeichnet,**
**dass** sie 2 bis 12 Gew.-% Peroxyessigsäure und 5 bis 30 Gew.-% Methansulfonsäure enthält und das Gewichtsverhältnis von Methansulfonsäure zu Peroxyessigsäure im Bereich von 2 bis 5 liegt.

## Claims

1. Method for producing equilibrium peracetic acid by reacting acetic acid with hydrogen peroxide in an aqueous reaction mixture, **characterized in that** the reaction is carried out in the presence of methanesulphonic acid as catalyst.

2. Method according to Claim 1, **characterized in that** the reaction mixture contains 0.1 to 2.0% by weight methanesulphonic acid.

3. Method according to Claim 1 or 2, **characterized in that** the reaction mixture does not contain additional solvent.

4. Method according to any one of Claims 1 to 3, **characterized in that** the reaction mixture is produced by mixing acetic acid, aqueous hydrogen peroxide solution having a content of 25 to 60% by weight hydrogen peroxide, and methanesulphonic acid, wherein the methanesulphonic acid can contain 1 to 40% by weight water.

5. Method according to any one of Claims 1 to 4, **characterized in that** acetic acid and hydrogen peroxide are used in a molar ratio in the range from 0.5 to 10.

6. Method according to any one of Claims 1 to 5, **characterized in that** the reaction is carried out until the content of peroxyacetic acid has reached more than 90% of the content present in the chemical equilibrium.

7. Use of methanesulphonic acid as catalyst for producing equilibrium peracetic acid from acetic acid and hydrogen peroxide.

8. Equilibrium peracetic acid comprising peroxyacetic acid, hydrogen peroxide, acetic acid and water, **characterized in that** it contains methanesulphonic acid.

9. Equilibrium peracetic acid according to Claim 8, **characterized in that** it contains 0 to 5% by weight surfactants, 0 to 10% by weight polymers containing carboxyl groups and less than 1% by weight additional compounds.

10. Equilibrium peracetic acid according to Claim 8 or 9, **characterized in that** it contains 3 to 17% by weight peroxyacetic acid.

11. Equilibrium peracetic acid according to Claim 8 or 9, **characterized in that** it contains 2 to 12% by weight peroxyacetic acid and 5 to 30% by weight methanesulphonic acid, and the weight ratio of methanesulphonic acid to peroxyacetic acid is in the range from 2 to 5.

## Revendications

1. Procédé pour la préparation d'acide peracétique à l'équilibre par transformation d'acide acétique avec du peroxyde d'hydrogène dans un milieu réactionnel aqueux, **caractérisé en ce que** la transformation est réalisée en présence d'acide méthanesulfonique comme catalyseur.

2. Procédé selon la revendication 1, **caractérisé en ce que** le mélange réactionnel contient 0,1 à 2,0% en poids d'acide méthanesulfonique.

3. Procédé selon la revendication 1 ou 2, **caractérisé en ce que** le mélange réactionnel ne contient pas de solvant supplémentaire.

4. Procédé selon l'une quelconque des revendications 1 à 3, **caractérisé en ce que** le mélange réactionnel est préparé par mélange d'acide acétique, d'une solution aqueuse de peroxyde d'hydrogène, présentant une teneur en peroxyde d'hydrogène de 25 à 60% en poids, et d'acide méthanesulfonique, l'acide méthanesulfonique pouvant contenir 1 à 40% en poids d'eau.

5. Procédé selon l'une quelconque des revendications 1 à 4, **caractérisé en ce que** l'acide acétique et le peroxyde d'hydrogène sont utilisés dans un rapport molaire de 0,5:10.

6. Procédé selon l'une quelconque des revendications 1 à 5, **caractérisé en ce que** la transformation est réalisée jusqu'à l'obtention d'une teneur en acide peroxyacétique supérieure à 90% de la teneur qui existe à l'équilibre chimique.

7. Utilisation d'acide méthanesulfonique comme catalyseur pour la préparation d'acide peracétique à l'équilibre à partir d'acide acétique et de peroxyde d'hydrogène.

8. Acide peracétique à l'équilibre comprenant de l'acide peroxyacétique, du peroxyde d'hydrogène, de l'acide acétique et de l'eau, **caractérisé en ce qu'**il contient de l'acide méthanesulfonique.

9. Acide peracétique à l'équilibre selon la revendication 8, **caractérisé en ce qu'**il contient 0 à 5% en poids de tensioactifs, 0 à 10% en poids de polymères contenant des groupes carboxyle et moins de 1% en poids de composés supplémentaires.

10. Acide peracétique à l'équilibre selon la revendication 8 ou 9, **caractérisé en ce qu'**il contient 3 à 17% en poids d'acide peroxyacétique.

11. Acide peracétique à l'équilibre selon la revendication 8 ou 9, **caractérisé en ce qu'**il contient 2 à 12% en poids d'acide peroxyacétique et 5 à 30% en poids d'acide méthanesulfonique et le rapport pondéral d'acide méthanesulfonique à acide peroxyacétique se situe dans la plage de 2 à 5.
